# EUROPEAN PATENT SPECIFICATION

(11) **EP 3 331 437 B1**
(45) Date of publication and mention of the grant of the patent: **22.05.2019**
(21) Application number: 16741620.5
(22) Date of filing: 20.07.2016
(51) Int. Cl.: A61B 8/06, A61B 5/06, A61B 5/00, A61B 8/12, A61B 8/00, A61B 5/0285, A61B 8/08, A61B 5/026

(54) **CURVED SENSORIZED CATHETER**
GEKRÜMMTER SENSORISIERTER KATHETER
CATHÉTER À CAPTEUR INCURVÉ

(30) Priority: 03.08.2015 DE 102015010064
(43) Date of publication of application: 13.06.2018
(73) Proprietor: Medyria AG, 8406 Winterthur (CH)
(72) Inventor: SETTE, Mauro, 4500 Solothurn (CH)
(74) Representative: Frenkel, Matthias Alexander
(86) International application number: PCT/EP2016/067275
(87) International publication number: WO 2017/021146

(56) References cited:
- WO-A1-2014/186912
- US-A- 5 855 577
- US-A1- 2007 093 703

## Description

### Technical field

The present disclosure generally relates to the field of catheter technology. More specifically, the present disclosure relates to a catheter and to a catheter system comprising a catheter and a medical device to be introduced into the catheter.

### Background

In medicine, catheters serve a broad range of functions. In general, catheters are medical devices that can be inserted into the body to treat diseases or perform a surgical procedure. To give a few examples, catheters may be used for cardiovascular, urological, gastrointestinal, neurovascular, and ophthalmic applications, to name some examples.

Catheters can be inserted into a body cavity, duct, or vessel. Functionally, they allow drainage, administration of fluids or gases, access by surgical instruments, and also perform a wide variety of other tasks depending on the type of catheter. The process of inserting a catheter is often called catheterization. In general, a catheter is a thin tube made from medical grade materials. In most uses, a catheter is a thin, flexible tube ("soft" catheter) though catheters are available in varying levels of stiffness depending on the application. A catheter left inside the body, either temporarily or permanently, may be referred to as an indwelling catheter.

There is a class of surgical procedures called interventional procedures or minimal invasive procedures, which foresees the introduction of a catheter within the human vasculature. The catheter is used to deploy a device, make measurements, perform treatment and/or inject a fluid. In common procedures the catheter is introduced from an opening in a blood vessel, e.g. in the groin or in the carotids. Through this opening the catheter is then advanced until the region of interest, and the intervention is then performed at the region of interest. The intervention performed may be one or more of deploy a device, perform a treatment or measurement, and/or inject fluid.

Sometimes catheters are used for guiding other medical devices such as guide wires (often also called guidewires), balloons, or other catheters to certain anatomical structures such as side branches, heart valves, vessel's aneurisms, or vessel's bifurcations. For this purpose, the tip of the distal part of the catheter is normally approached to the anatomical structure, e.g. a side branch, and when the catheter's tip hits the structure the second device, e.g. a guide wire, is approached. Hitting the structure can be traumatic due to the sharp edged tip of the catheter, thus very often the catheter's tip is built with a softer material that makes it less traumatic.

In general the catheter's position is visualized by means of x-ray based machines. X-rays are ionizing radiations potentially harmful for the patients and the doctors. Thus, there this is a constant need and trend to reduce its use to a minimum. Moreover, the use of x-rays is often coupled with the use of a contrast dye. The contrast dye is a iodinated solution that is injected into the vessel under examination. The use of contrast dye makes it possible to enhance visibility of some important structures with vessels such as the side branches. The contrast dye is not free from side effect, its use induces sufferance in other organs, e.g. the kidneys, and depending on the patient's pathological status it can lead to permanent kidney impairment.

As stated above, the state of the art in identification of anatomical structures makes use of radiological tools such as contrast dye and x-ray. Contrast dye is injected into the vessel using a so called pig-tail or angiographic catheter. An x-ray image is created displaying the high contrast vessel. Different imaging techniques, like so called fluoro-overlay techniques are used in order to reproduce a pre-recorder image. This technique is used for limiting the use of contrast dye, but as soon as something changes in the image the method does not any more accurately represent the reality.

An alternative method for the identification of the anatomical structures is the use of a so-called Intravascular Ultrasound (IVUS). IVUS is a medical imaging methodology using a catheter with a miniaturized ultrasound probe attached to the catheter, e.g., to the distal end of the catheter. The proximal end of the catheter is normally attached to computerized ultrasound equipment. It allows the application of ultrasound technology, such as piezoelectric transducer or capacitive micromachined ultrasonic transducers (CMUT), to see from inside blood vessels out through the surrounding blood column, visualizing the endothelium (inner wall) of blood vessels in living individuals. To give an example, the arteries of the heart (the coronary arteries) are the most frequent imaging target for IVUS.

A catheter equipped with an IVUS image sensor is used for creating ultrasound-like images of the vessel's section. When a side branch is departing from the main vessel it can be identified on the image. The problem related to this technique is that side branches are not identified easily on the image and a very long training is required for the correct reading of the images. Moreover, there is no possibility of creating a frontal view.

The state of the art for the insertion of a guide wire or a catheter into a side branch uses most of the times a completely passive system. The passive system is often a catheter with a particular shape of the tip or body. Amplatz, Bernstein, and SOS Omni catheters are examples for passive systems, to cite some of them. One specific passive catheter is disclosed in US 8,562,573 B1. Several techniques might be used with passive catheters such as (i) catheter in catheter technique, and (ii) no touch technique. In those techniques a combination of different passive catheters is used. IVUS imaging techniques are sometimes used in combination with the passive catheters to overcome the problem of x-ray and contrast dye.

As stated above, current techniques for the identification of side branches make use of an external imaging system such as x-ray coupled with contrast dye or an intravascular imaging system such as IVUS. X-ray imaging is based on ionizing radiations which can trigger the formation of cancer, contrast-dye can induce renal insufficiency or allergic reactions, which limits the extensive use thereof. Both systems keep the imaging function separate from the guiding function. Even the IVUS imaging system cannot be integrated into a guiding catheter because it is supposed to be at a certain distance from the vessel's wall. Otherwise the side branch cannot be identified. Moreover, in approaching anatomical structures that are "in front" of the catheter IVUS cannot be used because it can provide only lateral images.

Document WO 2014/186912 A1 discloses a flow sensor arrangement located on an elongated body for measuring the flow of a fluid, with one or more flow sensors being arranged on the elongated body to measure the flow along different spatial directions.

Document US 5 855 577 A discloses a bow shaped, integrated wire catheter apparatus for using laser treatment within cavities and organs of the human body. The apparatus comprises a main catheter shaft having at least one hollow lumen, an outer arcuate sidewall and an inner arcuate sidewall. The outer arcuate sidewall has one or more guide holes disposed thereon for selectively providing laser on a selected surface by controllably advancing a laser delivery device through the one or more guide holes to the selected surface.

Engaging of the anatomical structures with guiding catheters should be performed by mechanical interaction between the sharp catheter's tip and the anatomy, which often leads to damages, such as damage to the intima vessel layer, dissections, detaching of atherosclerotic plaques, and detaching calcifications. For this reason the catheters used should have an a-traumatic tip for minimizing the risk of damaging the vessel. This complicates the catheter control even further, as the tip is normally not steerable like the catheter.

### Summary

Accordingly, there is a need for improved catheters.

According to a first aspect, a catheter according to independent claim 1 is provided. The catheter comprises an elongated body, a lumen, and one or more sensors. The elongated body comprises a distal tip. The lumen is arranged within the elongated body. The lumen comprises a lumen opening. The lumen opening is arranged at the elongated body. The one or more sensors are laterally arranged at the elongated body. The elongated body comprises an at least partially curved portion. At least one of the one or more sensors is arranged at the at least partially curved portion.

The elongated body may be or comprise an elongated tube. The elongated tube may have different degrees of flexibility or stiffness. For example, the elongated tube may be a flexible tube.

The one or more sensors may respectively comprise or be configured as at least one of one or more flow sensors and one or more ultrasound sensors. In consequence, one or more flow sensors may be laterally arranged at the elongated body. Alternatively or additionally, one or more ultrasound sensors may be laterally arranged at the elongated body. To give an example, the one or more ultrasound sensors may respectively comprise or be configured as an IVUS sensor. As described above, IVUS is a medical imaging methodology using a catheter with a miniaturized ultrasound probe attached to the catheter. The catheter may then be further attached to computerized ultrasound equipment that allows the application of ultrasound technology, such as piezoelectric transducer or Capacitive micromachined ultrasonic transducers (CMUT).

The one or more flow sensors may comprise or be configured as one or more flow velocity sensors for measuring a flow quantity such as the velocity of a fluid or medium in a vessel. For example, the one or more flow sensors may comprise or be configured as one or more thermo-convective or thermal flow or thermo-conductive sensors. The one or more flow sensors may respectively comprise a metallic resistance or thin film or thin plate or thermocouple. The one or more flow sensors may respectively be configured as wire anemometer sensors. For example, the full velocity vector of a flow can be determined by the one or more flow sensors in magnitude, direction and sense. For details regarding the flow sensors it is referred to International patent application CH2014/000068 (published as WO2014/186912) of the applicant, the content of which is incorporated by reference herewith.

The one or more flow sensors may comprise or be configured as one or more doppler sensors. To give one specific example, the one or more sensors may comprise or be configured as one or more ultrasound doppler flow sensors. Ultrasound doppler flow sensors can be summarized under "flow sensors", for example und "doppler sensors", as well as under "ultrasound sensors".

Further details and embodiments will in the following be described with respect to one or more flow sensors as an example of the one or more sensors that are arranged at the elongated body. The sole reference to one or more flow sensors is only used for purpose of explanation rather than limitation. That is, even if in the below it is referred only to one or more flow sensors, other types of sensors such as ultrasound sensors may be used in addition or instead.

The at least partially curved portion may be understood as a portion of the elongated body having one or more bent or curved portions. In other words, at least a part of the at least partially curved portion may be bent or curved with respect to a straight portion of the elongated body of the catheter. Independent of the exact realization of the at least partially curved portion, at least one of the one or more flow sensors is arranged at the least partially curved portion. Any number, e.g., all of the one or more flow sensors may be arranged at the at least partially curved portion.

The lumen opening may comprise or be configured as a lateral lumen opening. The lateral lumen opening may be laterally arranged at the elongated body proximal to the distal tip of the elongated body. In other words, if configured as a lateral lumen opening, the lateral lumen opening is not arranged at the distal tip of the elongated body. Rather, the lateral lumen opening may be distanced from the distal tip and arranged at a lateral location of the elongated body. In respect of catheters, the proximal part is normally the section of the catheter that remains outside the body, while distal is the opposite, i.e. the distal part refers to the section of the catheter that is (first) inserted into the body. In consequence, the distal tip is the tip of the catheter which is first introduced into the body.

The lumen opening may be arranged at the at least partially curved portion.

The at least one of the one or more flow sensors is arranged, in the longitudinal direction of the elongated body, at the same position as the lumen opening. The at least one of the one or more flow sensors is arranged, in circumferential direction of the elongated body, at a different position than the lumen opening.

The at least partially curved portion may comprise a straight section. The lumen opening may be arranged at the straight section of the at least partially curved portion. Alternatively or additionally, the at least one of the one or more flow sensors may be arranged at the straight section of the at least partially curved portion.

The at least partially curved portion may comprise a rounded section. The lumen opening may be arranged at the rounded section of the at least partially curved portion. Alternatively or additionally, the at least one of the one or more flow sensors may be arranged at the rounded section of the at least partially curved portion. The rounded section may be part of, may be or may comprise at least one of the one or more curved or bent portions of the at least partially curved portion.

The elongated body may comprise a straight portion. The lumen opening may be arranged at a position that is distanced from the straight portion in a direction perpendicular to the longitudinal direction of the straight portion. The straight portion may be a part of the elongated body that is different to the at least partially curved portion. For example, the straight portion and the at least partially curved portion may form the elongated body.

The catheter may comprise an adjusting component configured to adjust the shape of the at least partially curved portion, e.g., a radius or curvature of the at least partially curved portion. The adjusting component may comprise one or more wires that can be operated by means of an operating component.

The catheter may comprise at least one second lumen within the elongated body. The second lumen may comprise a lumen opening. The lumen opening of the second lumen may be arranged at the distal tip of the elongated body. Alternatively, the lumen opening of the second lumen may be arranged at a lateral position of the elongated body distanced from the distal tip of the elongated body.

The catheter may comprise a slide within the lumen. The slide may be configured to extend towards the lumen opening. By means of the slide a medical device inserted within the catheter, can be guided to the lumen opening, e.g., the lateral lumen opening. The slide may be configured and arranged to guide or push the device that is inserted into the lumen towards the lumen opening, e.g. the lateral lumen opening / side hole. In general, a device introduced into the lumen tends to extend in a straight manner. The slide may be configured to guide, e.g. bend, the device laterally. To give an example, the lumen may have the lateral lumen opening / side hole as the only opening through which the inserted medical device can exit. In that case the guiding, by the slide, of the medical device towards this opening / hole will be the only possibility for the medical device to exit. Further, for example, a first medical device may be inserted into the catheter and may be accommodated within the lumen, and a second medical device may be inserted into the catheter and may be accommodated within the lumen. The slide may guide the first medical device to the lateral lumen opening so that the first medical device exits through the lateral lumen opening. The second medical device may extend outside of the slide but within the catheter, e.g. within the lumen of the catheter, and may be advanced to another lumen opening such as an opening at the distal tip of the catheter. Further, for example, the slide may be configured and arranged such that a medical device may be inserted over a guidewire. Once the guidewire is removed or withdrawn, a catheter with bigger diameter may approach that will be pushed laterally by the slide.

The at least one of the one or more flow sensors may be configured to determine the location of an anatomical region or structure. The anatomical region or structure may comprise or be a side branch of a vessel, a stenosis of a vessel, or a heart valve. The anatomical region or structure is not limited to the aforementioned examples. For example, certain quantities detected by the one or more flow sensors may be interpreted by a processing unit in such a way that a side branch or a vessel bifurcation or other features of the vessel or a location thereof is identified. The location can be determined relative to the catheter. The one or more flow sensors may be configured to determine the flow by the magnitude of the fluid's flow only, that is, without information about the sign. Further, given information about the angular orientations of the one or more flow sensors, vector components of a flow vector can be computed. For example, a flow direction sign evaluation unit may be connected with the one or more flow sensors to determine, from signals of the one or more flow sensors, the direction of the flow according to measurement signals received from the one or more flow sensors. This may be derived from a difference in flow values determined from two or more of the one or more flow sensors, for example, wherein the flow sensor that returns the lower flow value may indicate the direction in which the flow is heading. Further, the flow direction sign evaluation unit may be connected with the one or more flow sensors to determine, from signals of the one or more flow sensors, the sign of the flow. The flow direction sign evaluation unit may be configured to not only determine the direction of the flow, but also the sign or sense of the flow in the direction(s) along the sensor(s).

The at least one of the one or more flow sensors may be configured to detect blood flow changes in a vessel into which the catheter is inserted. The at least one of the one or more flow sensors may be configured to derive the location of an anatomical region or structure from the detected blood flow changes.

The catheter may comprise a control unit. The control unit may be configured to determine a location and/or positioning of the catheter at which the lumen opening is aligned with the location of an anatomical region or structure based on an output of the at least one of the one or more flow sensors. To give a few examples, the anatomical region or structure may comprise or be a side branch of a vessel, a stenosis of a vessel, or a heart valve. For example, the control unit may be configured to determine the location and/or positioning of the catheter at which the lumen opening is aligned with the location of an orifice of a side branch of a vessel or at which the lumen opening faces the center of a heart valve based on an output of the at least one of the one or more flow sensors.

When the catheter is introduced into a vessel, the portion of the catheter on which the lumen opening is arranged may be deviated from the center of the vessel in a direction perpendicular to the longitudinal direction of the vessel. Alternatively or additionally, the portion of the catheter on which the at least one of the one or more flow sensors is arranged may be deviated from the center of the vessel in a direction perpendicular to the longitudinal direction of the vessel.

By means of the catheter as described herein, when bringing the at least partially curved portion close to an anatomical region or structure, the risk of damaging the region or structure or causing any trauma is significantly reduced as compared to bringing the distal tip close to the anatomical region or structure.

According to a second aspect, a catheter system is provided. The catheter system comprises the catheter according to the first aspect as described herein. The catheter system further comprises a medical device that is configured to be introduced into the lumen of the catheter. In general, any one of the details of the catheter according to the first aspect described herein may equally be embodied in the catheter system. The invention is defined by appended claims 1-14.

### Brief description of the drawings

In the following, the present disclosure will further be described with reference to exemplary embodiments illustrated in the Figures, in which:
- Figure 1: is a schematic illustration of a prior art catheter;
- Figure 2: is a schematic illustration of another prior art catheter;
- Figure 3a: is a schematic illustration of a catheter according to a first embodiment;
- Figure 3b: is a schematic illustration of the catheter according to the first embodiment at a different position;
- Figure 4: is a schematic illustration of a catheter according to a second embodiment;
- Figure 5: is a schematic illustration of a catheter according to a third embodiment;
- Figure 6: is a schematic illustration of the catheter according to the first embodiment of Figures 3a and 3b;
- Figure 7: is a schematic illustration of a variant of the catheter according to the first embodiment of Figures 3a and 3b;
- Figure 8: is a schematic illustration of the catheter according to the first embodiment of Figures 3a and 3b;
- Figure 9: is a schematic illustration of a flow mapping in the region of a side branch;
- Figure 10: is a flow mapping of an anatomical mockup; and
- Figure 11: is a schematic illustration of a diagram of measured flow velocity.

### Detailed description

In the following description, for purposes of explanation and not limitation, specific details are set forth, such as specific catheter shapes or sensor details, in order to provide a thorough understanding of the present disclosure. It will be apparent to one skilled in the art that the present disclosure may be practiced in other embodiments that depart from these specific details. For example, even if the present disclosure is described with reference to specific catheter shapes or sensors, the present disclosure may be practiced with other catheter shapes or sensors. That is, even if in the below it is referred only to one or more flow sensors, other types of sensors such as ultrasound sensors may be used in addition or instead.

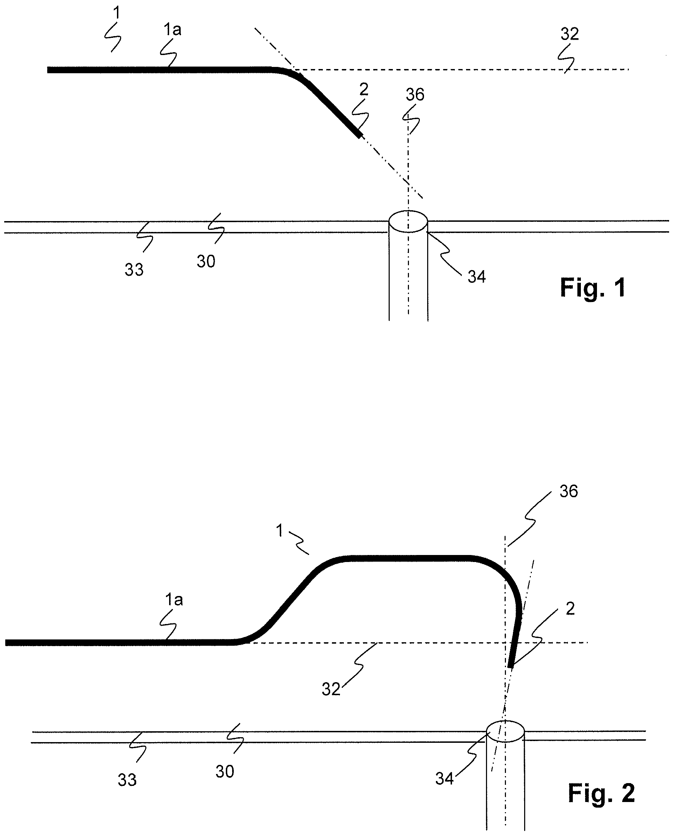

Figure 1 shows a schematic illustration of a prior art catheter 1. The prior art catheter 1 shown in Figure 1 is often referred to as Bernstein catheter. The catheter 1 is of a bent tip type, as its distal end portion close to the distal tip 2 is bent. In the example of figure 1, the catheter 1 is introduced into a vessel 30. In the example shown in figure 1, a straight potion 1a of the elongated body of the catheter 1 lies on the center 32 of the vessel 30 and the tip portion of the catheter 1 is bent towards the wall 33 of the vessel 30. Further, a side branch 34 of vessel 30 is shown in figure 1. The side branch 34 of the vessel 30 has a center 36. In order to perform a certain operation at or close to the side branch 34, the catheter 1 has to be brought close to the side branch 34, for example the distal tip 2 has to be aligned with the center 36 of the side branch 34. In consequence, the distal tip 2 of the catheter 1 may damage the side branch 34 or may cause any other trauma.

Figure 2 shows a schematic illustration of another prior art catheter 1. The catheter 1 is also introduced into a vessel 30 similar to the one shown in Figure 1. Thus, regarding the details of the vessel 30 and the side branch 34 it is referred to figure 1 above. As can be seen from figure 2, the catheter 1 is also bent. The distal tip 2 of catheter 1 has a certain angulation in order to engage the center 36 of side branch 34. However, when the distal tip 2 is getting close to or contacts the side branch 34, there is a risk of damaging the side branch 34 or causing any other trauma.

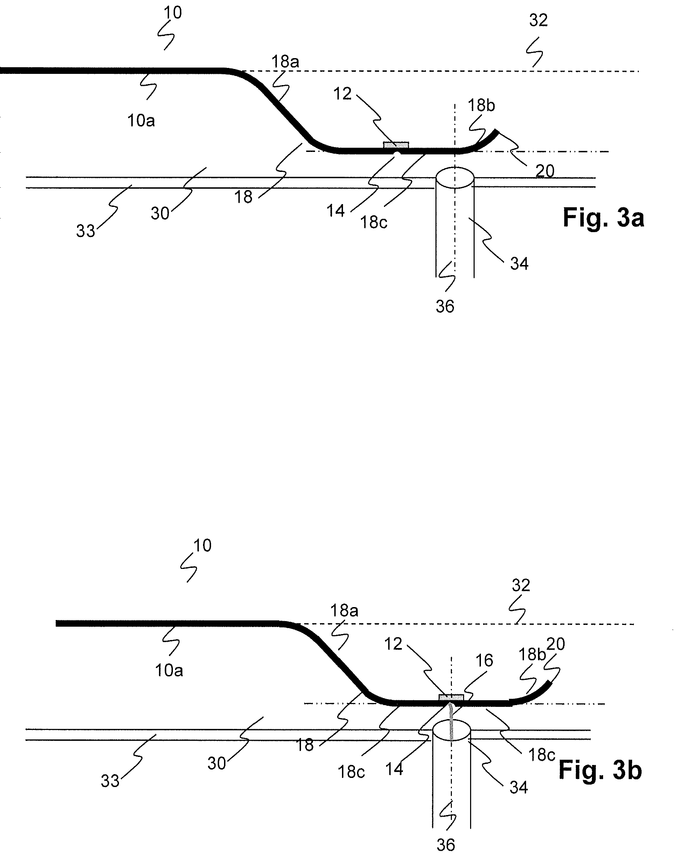

Figures 3a and 3b show a catheter 10 according to a first embodiment. The catheter 10 is also introduced into a vessel 30 similar to the ones shown in Figures 1 and 2. Thus, regarding the details of the vessel 30 and the side branch 34 it is referred to figures 1 and 2 above. The catheter 10 of figure 3a is identical to the catheter 10 of figure 3b, it is just positioned at a different location within the vessel in figures 3a and 3b.

Unlike the prior art catheters 1 of figures 1 and 2 the catheter 10 according to the first embodiment is equipped with a flow sensor 12. Although only one flow sensor 12 is shown in figures 3a and 3b, the catheter 10 may include any number of flow sensors 12, i.e. one or more flow sensors 12. In the following, it will only be referred to the flow sensor 12 without limiting the number of flow sensors 12 to any specific number. The flow sensor 12 may be a flow velocity sensor. For example, the flow sensor 12 may be configured as a thermo-convective sensor as will be described in more detail below. The catheter 10 further comprises an opening 14 or hole that is part of or connected to a lumen within the catheter 10. The opening 14 is therefore referred to as lumen opening 14. The lumen opening 14 is arranged at the side of the elongated body of the catheter, i.e. the opening 14 is arranged laterally at the elongated body. Thus, the lumen opening 14 may also be referred to as lateral lumen opening 14. There may be one or more openings 14 arranged at the catheter 10. A medical device 16 such as a guide wire, guide catheter, balloon catheter or the like may be introduced into the lumen of the catheter 12 and may exit from the catheter 10 through the lumen opening 14. In general, a catheter adapted for guiding a medical device, inserted into the catheter, to a certain location, may be referred to as guiding catheter.

The catheter 10 according to the first embodiment includes an at least partially curved portion 18 (that may also be referred to as at least partially bent portion). The at least partially curved portion 18 may be understood as a portion of the elongated body of the catheter 10 that is bent with respect to the straight portion 10a of the catheter 10 and that has one or more bent or curved portions or sections. In other words, at least a part of the at least partially curved portion 18 is bent or curved with respect to the straight portion 10a of the elongated body of the catheter 10. In the exemplary shape shown in figures 3a and 3b, the at least partially curved portion 18 comprises a first curved section 18a (bent section 18a) that is bent away from the straight portion 10a of the catheter 10 and a second curved section 18b (bent section 18b) that is bent back (in a direction back to the straight portion 10a of the catheter 10). A straight section 18c is arranged between the two curved sections 18a and 18b. Thus, in the exemplary configuration of figures 3a and 3b, the at least partially curved portion 18 comprises a first curved section 18a, a straight section 18c and a second curved section 18b. Further, in the exemplary configuration of figures 3a and 3b, the flow sensor 12 and the lumen opening 14 are arranged at the straight section 18c of the at least partially curved portion 18. The first curved section 18a and the second curved section 18b do not necessarily have to be curved along their whole length, as long as at least a part of the first curved section 18a and the second curved section 18b is curved such that the at least partially curved portion 18 is bent with respect to the straight portion 10a of the catheter 10.

The at least partially curved portion 18 of the catheter 10 having the flow sensor 12 is deviated from the center 32 of the vessel 30 in a direction perpendicular to the longitudinal direction of the straight portion 10a. Thus, it can bring the flow sensor 12 closer to the vessel's wall 33 without damaging the vessel's wall. Further, the opening 14 can be brought close to the side branch 34 without any sharp edge such as the tip of the catheter damaging the side branch 34. The flow sensor 12 can recognize when the center 36 of the side branch 34 is reached. In accordance therewith, the catheter 10 can be placed such that the flow sensor 12 and/or the lumen opening 14 is aligned with the center 36 of the side branch 34. The latter is shown in figure 3b. In other words, looking at the flow (velocity) signal determined by the flow sensor 12, it is possible to identify when the flow sensor 12 together with the opening 14 is aligned with the center 36 of the side branch orifice of the side branch 34.

Aligning the flow sensor 12 and/or the opening 14 with the center 36 of the side branch 34 may be achieved in various ways. For example, when the catheter 10 is moved in the proximity of the side branch 34 the flow changes. The change can be magnitude, direction or sense or a combination of the three quantities. Changes in the magnitude of the flow can be measured by the flow sensor 12 in that there is an increase when there is a striction and a decrease when there is a widening, e.g. in the case of the stenotic valve an increase of flow velocity is measured when approaching the leaflets' commissure. In the case of the side branch 34 the flow velocity direction deviates from a direction along the vessel's longitudinal direction to a direction more perpendicular to this first one. The sense changes in the direction of the side branch 34, i.e. left or right side branch.

Due to the shape of the catheter 10, the sensor 12 is brought in a region of the vessel where there is a difference between two flow velocities measured: when the sensor 10 is scanning the region close to the vessel's wall (Fig. 3a), and/or when it is facing the orifice of the side branch (Fig. 3b). This difference is used for the identification of the anatomical location.

In the exemplary configuration of figures 3a and 3b, the flow sensor 12 and the opening 14 are arranged at the same position along the elongated body of the catheter 10 but at a different position along the circumference of the elongated body. Thus, by aligning the flow sensor 12 with the center 36 of the side branch 34, the opening is automatically aligned with the center 36 of the side branch 34. If the flow sensor 12 and the opening are distanced from each other by a predetermined distance along the longitudinal direction of the elongated body of the catheter 10, the control unit may consider this predetermined distance in order to align the opening 14 with the center 36 of the side branch from measurement signals of the flow sensor 12.

As the lumen opening 14 is arranged at the at least partially curved portion 18, more particularly at the straight section 18c of the at least partially curved portion 18, the side branch 34 is not damaged when the catheter 10 approaches or is positioned close to the side branch 34 or even contacts the side branch 34.

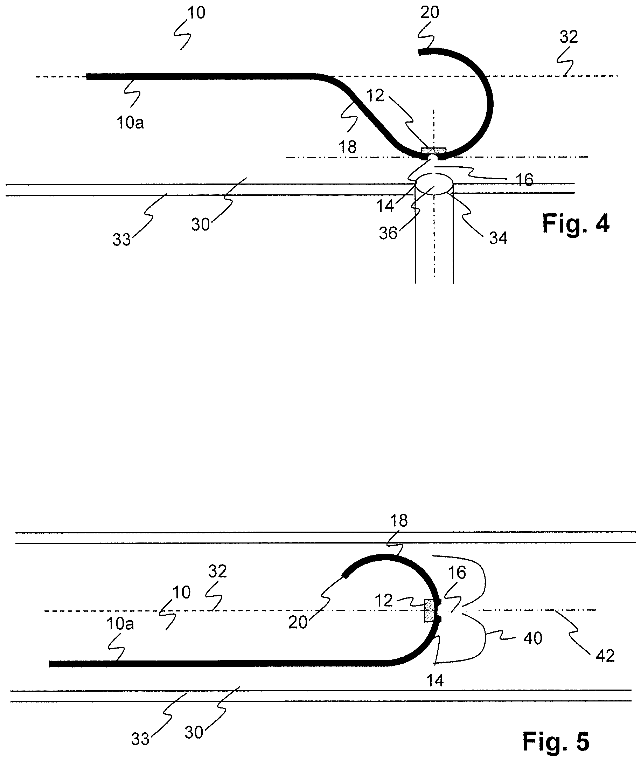

Figure 4 shows a schematic illustration of a catheter 10 according to a second embodiment. The catheter 10 is also introduced into a vessel 30 similar to the ones shown in Figures 1 to 3b. Thus, regarding the details of the vessel 30 and the side branch 34 it is referred to figures 1 to 3b above.

The catheter 10 according to the second embodiment of figure 4 only differs from the catheter 10 according to the first embodiment of figures 3a and 3b in the shape of the at least partially curved portion 18. All other details may correspond to each other or may at least be similar. The at least partially curved portion 18 of the catheter 10 according to the second embodiment is curved in an almost circular manner to form at least a half circle. The flow sensor 12 and the opening 14 are arranged at a round section of the at least partially curved portion 18. Other geometries and shapes than the one shown in figures 3a to 4 are possible for the catheter's geometry.

The at least partially curved portion 18 of the catheter 10 having the flow sensor 12 is deviated from the center 32 of the vessel 30 in a direction perpendicular to the straight portion 10a of the elongated body of the catheter 10. Thus, the flow sensor 12 and the opening 14 can be brought closer to the vessel's wall 33 without causing any damage, harm or trauma. The flow sensor 12 can recognize when the center 36 of the side branch 34 is reached. In accordance therewith, the catheter 10 can be placed such that the sensor 12 and/or the lumen opening 14 is aligned with the center 36 of the side branch 34. The latter is shown in figure 4. In other words, looking at the flow (velocity) signal determined by the flow sensor 12, it is possible to identify when the sensor 12 together with the opening 14 is aligned with the center 36 of the side branch orifice of the side branch 34. If the flow sensor 12 and the opening 14 are distanced from each other, this distance can be considered for aligning the opening 14 with the center 36 of the side branch 34 as mentioned above.

As the lumen opening 14 is arranged at the at least partially curved portion 18, more particularly at a rounded section of the at least partially curved portion 18, the side branch 34 is not damaged when the catheter 10 approaches or is positioned close to the side branch 34 or even contacts the side branch 34.

The flow sensor 12 of any of the embodiments described herein can provide deterministic information on the position of the anatomical location such as the side branch 34. This means that the catheter 10 does not have to engage mechanically with the anatomical location but instead, thanks to the sensor measurement(s), can be aligned to the opening of the side branch 34 facilitating the engagement with the opening. In this way, the anatomical position is identified not with a "trial an error process" trying to hit the target but with a more deterministic approach. Once the catheter 10 is correctly aligned with the side branch 34 another medical device can be advanced through the opening 14.

The same or a similar type of catheter 10 can be used also for identifying the center 42 of a heart valve 40 as shown in figure 5. The flow sensor 12 is able to identify when the catheter's opening 14 is facing the center 42 of the valve 40. In this way, a second device like a guide wire can be approached easily through the valve's leaflets.

Figure 5 shows a schematic illustration of a catheter 10 according to a third embodiment. The catheter 10 is also introduced into a vessel 30 similar to the ones shown in Figures 1 to 4. Thus, regarding the details of the vessel 30 it is referred to figures 1 to 4 above. In contrast to figures 3a to 4 above, an aortic valve 40 is shown and the lumen opening 14 is to be aligned with the center 42 of the valve 40.

The catheter 10 is equipped with a flow (velocity) sensor 12. By means of the flow (velocity) sensor 12 the higher flow coming from the center 42 of the aortic valve 40 can be found, being the valve stenotic or not. Once the opening 14 is aligned with the center 42 of the valve 40, a medical device can be approached out of the opening 14 to the valve.

The shape of the at least partially curved portion 18 is similar to the one of the catheter 10 according to the second embodiment shown in figure 4. However, the flow sensor 12 and the opening 14 are positioned at a different location on the at least partially curved portion 18.

As the lumen opening 14 is arranged at the at least partially curved portion 18, more particularly at a rounded section of the at least partially curved portion 18, the aortic valve 40 is not damaged when the catheter 10 approaches or is positioned close to the side branch 34 or even contacts the side branch 34.

Summarizing the above, the catheter 10 of all embodiments has an elongated body with a particular (at least partially curved) shape. In the exemplary examples of figures 3a to 5 this particular shape is provided at the distal part (proximal part is the section that remains outside the body, while distal is the opposite). The present disclosure is, however, not limited to the particular shape being at the distal part. The particular shape allows the sensor 12 to get close to the side wall 33 of the vessel 30 as shown in figures 3a to 4. A lateral lumen opening 12 is present on the catheter shaft, the lateral lumen opening 14 is connected to an inner lumen that is used to insert a medical device (e.g. a guide wire). In contrast to prior art catheters 1 in which the opening is placed on the distal tip 2, there is no sharp edge in contact with the vessel's wall 33 in the embodiments described with respect to figures 3a to 5, but only the catheter's body, with the result of being less traumatic.

The catheter 10 may comprise an adjusting component for adjusting the shape of the catheter, for example for adjusting the shape of the at least partially curved portion 18. For example, the catheter's curvature can be actively changed by means of the adjusting component. The adjusting component may comprise a system of wire. By operating the wires, the catheter section holding the sensor 12 may be placed closer or more distant from the side branch 34 or the aortic valve 40, for example.

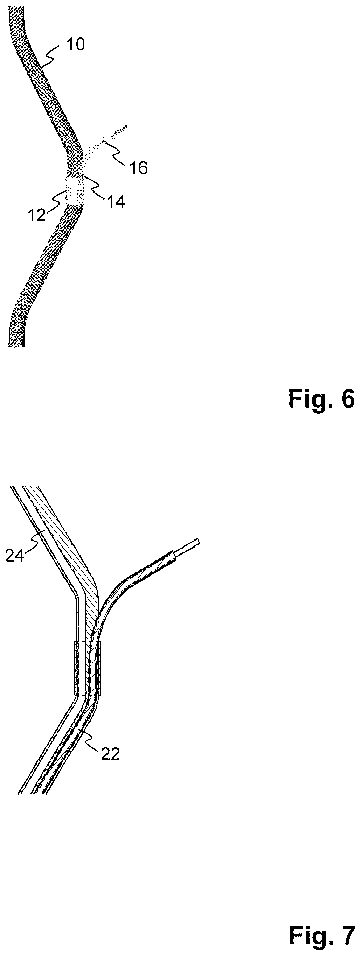

Figure 6 schematically illustrates a catheter system comprising a catheter 10 according to the first embodiment and a medical device 16. The catheter of figure 6 has a flow sensor 12 and an opening that can be used for approaching a second medical device. As can be seen in figure 6, the medical device 16 exits the catheter 10 at the lateral lumen opening 14 that is arranged laterally at the at least partially curved portion 18 of the catheter 10 according to the first embodiment.

Figure 7 schematically shows a variant of the catheter 10 according to the first embodiment. Figure 7 shows a catheter having two lumens 22, 24 for inserting two medical devices such as two guide wires or two different medical devices having, for example, different diameter. In other words, according to this variant, two lumens 22, 24 are arranged within the catheter 10. A first lumen is configured to accommodate a first medical device and the second lumen 24 is configured to accommodate a second medical device. The diameter of the cross-section through the first lumen 22 and the second lumen 24 may differ from each other. For example, the diameter of the cross-section of the first lumen 22 may be larger than the diameter of the cross-section of the second lumen 24. In the latter case, a medical device having a larger diameter may be introduced into the first lumen 22 and a medical device having a smaller diameter may be introduced into the second lumen 24. The first lumen 22 may have a lateral lumen opening 14 and the second lumen 24 may have a lumen opening at the distal end of the catheter 10. In order to guide a first medical device to the lateral lumen opening 14, the catheter 10 may include a slide inside the lumen with a hole in the center. The slide may be configured to guide the first medical device towards the lateral lumen opening 14 (side opening). Elements with smaller diameter (e.g. guide wires) can be advanced towards the distal opening of the catheter 10. In short, the catheter 10 shown in figure 7 may have two openings, one at the side and one distal, so that small diameters devices can be advanced towards the distal end, and bigger diameter devices are directed towards the side hole by the slide.

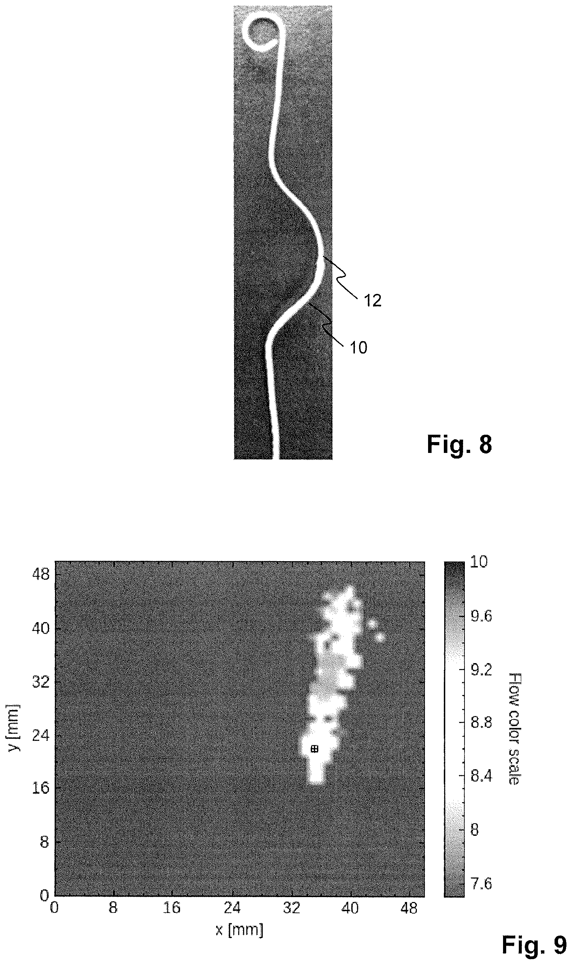

Figure 8 shows a photograph of a catheter 10 having a similar shape as the catheter 10 according to the first embodiment. The catheter 10 has a distal tip like a prior art catheter but the flow sensor 12 with the opening 14 placed on the side (laterally) as described above.

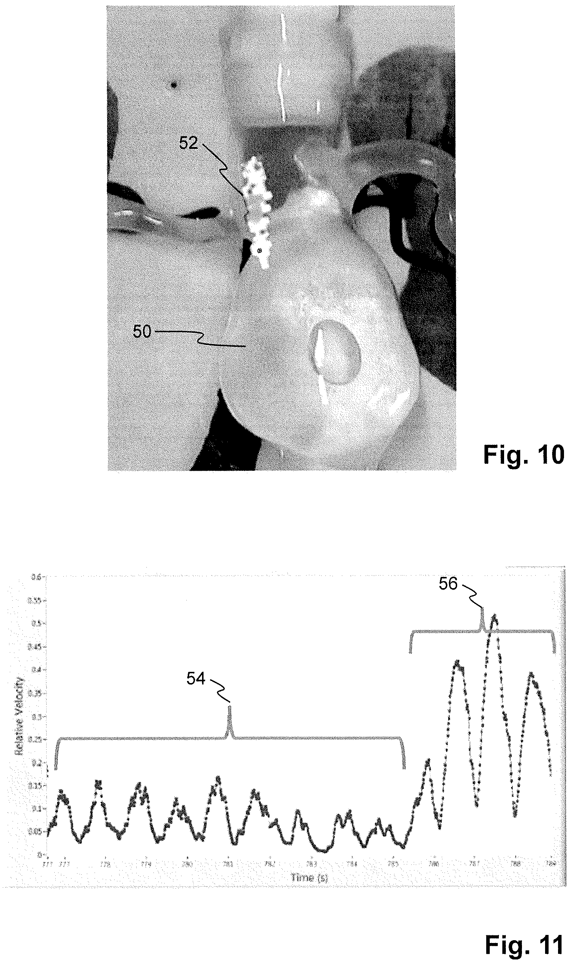

With respect to figures 9 to 11 some details of the flow sensor 12 are explained. Figure 9 shows flow mapping in the region of the side branch 34. Figure 10 shows flow mapping on anatomical mockup. It was demonstrated experimentally that the flow sensor 12 could identify the center 42 of a valve 40. Figure 11 shows flow velocity measured with a flow sensor 12. Flow velocity is higher when close to the anatomical structure. When the sensor 12 is far from the anatomical structure the flow velocity measured is lower (see section 54 in figure 11) and when the sensor 12 moves towards the anatomical location the flow velocity signal gets higher (see section 56 in figure 11). That is, blood's flow speed changes its pattern when approaching certain anatomical regions, such as side branches 34 or stenosis (e.g. valves 40). The catheter 10 equipped with flow (velocity) sensor(s) 12 can perform measurements as represented in figures 9 and 10 in form of flow map, and in figure 11 in form of velocity vs. time in order to enable a processing unit to derive the location of the anatomical region.

Certain anatomical structures can be identified by looking at the changes of the flow velocity, magnitude, direction and/or sense. This then facilitates the cannulation of catheters or delivery of certain medical devices. This further eliminates the need of using catheters with sharp edges.

By means of a catheter 10 equipped with one or more flow (velocity) sensor(s) 12 as described herein, the flow velocity or other quantities can be sensed / measured in different directions. The flow (velocity) sensor(s) 12 is / are able to sense changes in flow velocity / speed and flow direction, thereby being able to sense when the flow changes its profile that it normally has when flowing into a pipe / vessel. Strictions, stenosis and side branches 34 can then be identified.

Many advantages of the present disclosure will be fully understood from the foregoing description, and it will be apparent that various changes may be made in the form, construction and arrangement of the units and devices without departing from the scope of the present disclosure and/or without sacrificing all of its advantages. The invention is defined by claims 1-14.

## Claims

1. A catheter (10) comprising:
- an elongated body comprising a distal tip;
- a lumen arranged within the elongated body and comprising a lumen opening (14) arranged at the elongated body; and
- one or more sensors (12) laterally arranged at the elongated body;
wherein the elongated body comprises an at least partially curved portion (18) and at least one of the one or more sensors is arranged at the at least partially curved portion,
wherein the at least one of the one or more sensors is arranged, in the longitudinal direction of the elongated body, at the same position as the lumen opening and is arranged, in circumferential direction of the elongated body, at a different position than the lumen opening.

2. The catheter of claim 1, wherein the lumen opening comprises or is configured as a lateral lumen opening laterally arranged at the elongated body proximal to the distal tip of the elongated body.

3. The catheter of claim 1 or 2, wherein the lumen opening is arranged at the at least partially curved portion; and/or
wherein the one or more sensors comprise or are configured as at least one of one or more flow sensors and one or more ultrasound sensors.

4. The catheter of any one of claims 1 to 3, wherein the at least partially curved portion comprises a straight section (18c), and the lumen opening and/or the at least one of the one or more sensors is/are arranged at the straight section of the at least partially curved portion.

5. The catheter of any one of claims 1 to 4, wherein the at least partially curved portion comprises a rounded section, and the lumen opening and/or the at least one of the one or more sensors is/are arranged at the rounded section of the at least partially curved portion.

6. The catheter of any one of claims 1 to 5, wherein the elongated body comprises a straight portion (10a), and the lumen opening is arranged at a position that is distanced from the straight portion in a direction perpendicular to the longitudinal direction of the straight portion.

7. The catheter of any one of claims 1 to 6, wherein the catheter comprises an adjusting component configured to adjust the shape of the at least partially curved portion, e.g., a radius of the at least partially curved portion.

8. The catheter of any one of claims 1 to 7, wherein the catheter comprises at least one second lumen (22, 24) within the elongated body; and/or
wherein the catheter comprises a slide within the lumen, the slide being configured to extend towards the lumen opening.

9. The catheter of any one of claims 1 to 8, wherein the at least one of the one or more sensors is configured to determine the location of an anatomical region or structure (34).

10. The catheter of claim 9, wherein the anatomical region or structure comprises or is a side branch of a vessel, a stenosis of a vessel, or a heart valve.

11. The catheter of any one of claims 1 to 10, wherein the at least one of the one or more sensors is configured to detect blood flow changes in a vessel into which the catheter is inserted and to derive the location of an anatomical region or structure from the detected blood flow changes.

12. The catheter of any one of claims 1 to 11, wherein the catheter comprises a control unit, wherein the control unit is configured to determine a location and/or positioning of the catheter at which the lumen opening is aligned with the location of an anatomical region or structure based on an output of the at least one of the one or more sensors, for example the control unit is configured to determine the location and/or positioning of the catheter at which the lumen opening is aligned with the location of an orifice of a side branch of a vessel or at which the lumen opening faces the center of a heart valve based on an output of the at least one of the one or more sensors.

13. The catheter of any one of claims 1 to 12, wherein, when the catheter is introduced into a vessel, the portion of the catheter on which the lumen opening and/or the at least one of the one or more sensors is/are arranged is deviated from the center of the vessel in a direction perpendicular to the longitudinal direction of the vessel.

14. A catheter system comprising:
- the catheter of any one of claims 1 to 13; and
- a medical device that is configured to be introduced into the lumen of the catheter.

## Patentansprüche

1. Katheter (10), umfassend:
- einen langgestreckten Körper, der eine distale Spitze umfasst;
- ein Lumen, das in dem langgestreckten Körper angeordnet ist und eine Lumenöffnung (14) umfasst, die an dem langgestreckte Körper angeordnet ist; und
- einen oder mehrere Sensoren (12), die seitlich am langgestreckten Körper angeordnet sind;
wobei der langgestreckte Körper einen zumindest teilweise gekrümmten Abschnitt (18) umfasst, und mindestens einer von dem einen oder den mehreren Sensoren an dem zumindest teilweise gekrümmten Abschnitt angeordnet ist,
wobei der mindestens eine von dem einen oder den mehreren Sensoren in der Längsrichtung des langgestreckten Körpers an der gleichen Position wie die Lumenöffnung angeordnet ist, und in Umfangsrichtung des langgestreckten Körpers an einer von der Lumenöffnung verschiedenen Position angeordnet ist.

2. Katheter nach Anspruch 1, wobei die Lumenöffnung eine laterale Lumenöffnung umfasst oder als eine solche konfiguriert ist, die seitlich am langgestreckten Körper proximal zur distalen Spitze des langgestreckten Körpers angeordnet ist.

3. Katheter nach Anspruch 1 oder 2, wobei die Lumenöffnung an dem zumindest teilweise gekrümmten Abschnitt angeordnet ist; und/oder
wobei der eine oder die mehreren Sensoren mindestens einen von einem oder mehreren Durchflusssensoren und einem oder mehreren Ultraschallsensoren umfassen oder als solche konfiguriert sind.

4. Katheter nach einem der Ansprüche 1 bis 3, wobei der zumindest teilweise gekrümmte Abschnitt einen geraden Abschnitt (18c) umfasst, und die Lumenöffnung und/oder der mindestens eine von dem einem oder den mehreren Sensoren am geraden Abschnitt des zumindest teilweise gekrümmten Abschnitts angeordnet ist/sind.

5. Katheter nach einem der Ansprüche 1 bis 4, wobei der zumindest teilweise gekrümmte Abschnitt einen gerundeten Abschnitt umfasst, und die Lumenöffnung und/oder der mindestens eine von dem einen oder den mehreren Sensoren am gerundeten Abschnitt des zumindest teilweise gekrümmten Abschnitts angeordnet ist/sind.

6. Katheter nach einem der Ansprüche 1 bis 5, wobei der langgestreckte Körper einen geraden Abschnitt (10a) umfasst und die Lumenöffnung an einer Position angeordnet ist, die vom geraden Abschnitt in einer zur langgestreckten Richtung des geraden Abschnitts rechtwinkligen Richtung beabstandet ist.

7. Katheter nach einem der Ansprüche 1 bis 6, wobei der Katheter eine Einstellkomponente umfasst, die zum Einstellen der Form des zumindest teilweise gekrümmten Abschnitts konfiguriert ist, beispielsweise eines Radius des zumindest teilweise gekrümmten Abschnitts.

8. Katheter nach einem der Ansprüche 1 bis 7, wobei der Katheter mindestens ein zweites Lumen (22, 24) in dem langgestreckten Körper umfasst; und/oder wobei der Katheter einen Schieber in dem Lumen umfasst, wobei der Schieber so konfiguriert ist, dass er sich zur Lumenöffnung erstreckt.

9. Katheter nach einem der Ansprüche 1 bis 8, wobei der mindestens eine des einen oder der mehreren Sensoren zum Feststellen des Orts eines anatomischen Bereichs oder einer anatomischen Struktur (34) konfiguriert ist.

10. Katheter nach Anspruch 9, wobei der anatomische Bereich oder die anatomische Struktur einen Seitenzweig eines Gefäßes, eine Stenose eines Gefäßes oder eine Herzklappe umfasst oder ein(e) solche(r) ist.

11. Katheter nach einem der Ansprüche 1 bis 10, wobei der mindestens eine von dem einen oder den mehreren Sensoren zum Feststellen von Blutflussveränderungen in einem Gefäß, in das der Katheter eingeführt ist, und zum Ableiten des Orts eines anatomischen Bereichs oder einer anatomischen Struktur aus den festgestellten Blutflussänderungen konfiguriert ist.

12. Katheter nach einem der Ansprüche 1 bis 11, wobei der Katheter eine Steuereinheit umfasst, wobei die Steuereinheit zum Feststellen, aufgrund einer Ausgabe von dem mindestens einen des einen oder der mehreren Sensoren, eines Orts und/oder einer Position des Katheters konfiguriert ist, an dem/der die Lumenöffnung mit dem Ort eines anatomischen Bereichs oder einer anatomischen Struktur abgestimmt ist, beispielsweise ist die Steuereinheit zum Feststellen, aufgrund einer Ausgabe von dem mindestens einen des einen oder der mehreren Sensoren, des Orts und/oder der Position des Katheters konfiguriert, an dem/der die Lumenöffnung mit dem Ort einer Öffnung eines Seitenzweigs eines Gefäßes abgestimmt ist, oder an dem die Lumenöffnung der Mitte einer Herzklappe gegenüberliegt.

13. Katheter nach einem der Ansprüche 1 bis 12, wobei, wenn der Katheter in ein Gefäß eingeführt wird, der Abschnitt des Katheters, an dem die Lumenöffnung und/oder der mindestens eine von dem einen oder den mehreren Sensoren angeordnet sind, von der Mitte des Gefäßes in einer zur Längsrichtung des Gefäßes rechtwinkligen Richtung abweicht.

14. Kathetersystem, umfassend:
- den Katheter nach einem der Ansprüche 1 bis 13; und
- eine medizinische Vorrichtung, der zum Einführen in das Lumen des Katheters konfiguriert ist.

## Revendications

1. Cathéter (10) comprenant :
- un corps allongé comprenant une pointe distale ;
- une lumière ménagée à l'intérieur du corps allongé et comprenant une ouverture de lumière (14) située sur le corps allongé ; et
- un ou plusieurs capteurs (12) disposés latéralement sur le corps allongé ;
le corps allongé comprenant une partie au moins partiellement incurvée (18) et au moins un parmi le ou les capteurs étant disposé à la partie au moins partiellement incurvée,
l'au moins un parmi le ou les capteurs étant disposé, dans la direction longitudinale du corps allongé, dans la même position que l'ouverture de lumière et étant disposé, dans une direction circonférentielle du corps allongé, dans une position différente de l'ouverture de lumière.

2. Cathéter selon la revendication 1, dans lequel l'ouverture de lumière comprend ou est configurée sous la forme d'une ouverture de lumière latérale ménagée latéralement sur le corps allongé de manière proximale à la pointe distale du corps allongé.

3. Cathéter selon la revendication 1 ou 2, dans lequel l'ouverture de lumière est ménagée sur la partie au moins partiellement incurvée ; et/ou
le ou les capteurs comprennent ou sont configurés sous la forme d'au moins un parmi un ou plusieurs capteurs de débit et un ou plusieurs capteurs ultrasonores.

4. Cathéter selon l'une quelconque des revendications 1 à 3, dans lequel la partie au moins partiellement incurvée comprend une section droite (18c), et l'ouverture de lumière et/ou l'au moins un parmi le ou les capteurs est/sont disposés à la section droite de la partie au moins partiellement incurvée.

5. Cathéter selon l'une quelconque des revendications 1 à 4, dans lequel la partie au moins partiellement incurvée comprend une section arrondie, et l'ouverture de lumière et/ou l'au moins un parmi le ou les capteurs est/sont disposés à la section arrondie de la partie au moins partiellement incurvée.

6. Cathéter selon l'une quelconque des revendications 1 à 5, dans lequel le corps allongé comprend une partie droite (10a), et l'ouverture de lumière est ménagée dans une position qui est à distance de la partie droite dans une direction perpendiculaire à la direction longitudinale de la partie droite.

7. Cathéter selon l'une quelconque des revendications 1 à 6, le cathéter comprenant un composant d'ajustement configuré pour ajuster la forme de la partie au moins partiellement incurvée, par exemple, un rayon de la partie au moins partiellement incurvée.

8. Cathéter selon l'une quelconque des revendications 1 à 7, le cathéter comprenant au moins une seconde lumière (22, 24) à l'intérieur du corps allongé ; et/ou le cathéter comprenant un coulisseau à l'intérieur de la lumière, le coulisseau étant configuré pour s'étendre vers l'ouverture de lumière.

9. Cathéter selon l'une quelconque des revendications 1 à 8, dans lequel l'au moins un parmi le ou les capteurs est configuré pour déterminer l'emplacement d'une région ou structure anatomique (34).

10. Cathéter selon la revendication 9, dans lequel la région ou structure anatomique comprend ou est une branche latérale d'un vaisseau, une sténose d'un vaisseau ou une valve cardiaque.

11. Cathéter selon l'une quelconque des revendications 1 à 10, dans lequel l'au moins un parmi le ou les capteurs est configuré pour détecter des changements de débit sanguin dans un vaisseau dans lequel le cathéter est introduit et pour déduire l'emplacement d'une région ou structure anatomique à partir des changements de débit sanguin détectés.

12. Cathéter selon l'une quelconque des revendications 1 à 11, le cathéter comprenant une unité de commande, l'unité de commande étant configurée pour déterminer un emplacement et/ou un positionnement du cathéter auquel l'ouverture de lumière est alignée avec l'emplacement d'une région ou structure anatomique sur la base d'une sortie de l'au moins un parmi le ou les capteurs, par exemple l'unité de commande étant configurée pour déterminer l'emplacement et/ou le positionnement du cathéter auquel l'ouverture de lumière est alignée avec l'emplacement d'un orifice d'une branche latérale d'un vaisseau ou auquel l'ouverture de lumière fait face au centre d'une valve cardiaque sur la base d'une sortie de l'au moins un parmi le ou les capteurs.

13. Cathéter selon l'une quelconque des revendications 1 à 12, dans lequel, lorsque le cathéter est introduit dans un vaisseau, la partie du cathéter sur laquelle l'ouverture de lumière et/ou l'au moins un parmi le ou les capteurs est/sont disposés, est déviée du centre du vaisseau dans une direction perpendiculaire à la direction longitudinale du vaisseau.

14. Système de cathéter comprenant :
- le cathéter selon l'une quelconque des revendications 1 à 13 ; et
- un dispositif médical qui est configuré pour être introduit dans la lumière du cathéter.
